# EUROPEAN PATENT APPLICATION

(11) **EP 3 795 347 A1**
(43) Date of publication of application: **24.03.2021**
(21) Application number: 19803167.6
(22) Date of filing: 26.04.2019
(51) Int. Cl.: B32B 7/022, A61L 15/16, A61L 15/24, A61L 15/26, A61L 15/28, A61L 15/32, A61L 15/40, A61L 15/42, A61L 15/44, B32B 27/12, B32B 33/00

(54) **TRANSFER SHEET, AND METHOD FOR TRANSFERRING THIN FILM LAYER**

(30) Priority: 16.05.2018 JP 2018094543
(71) Applicant: Toppan Printing Co., Ltd., Tokyo 110-0016 (JP)
(72) Inventor: MORISHIMA, Natsumi, Tokyo 110-0016 (JP); SAKAIRI, Koji, Tokyo 110-0016 (JP)
(74) Representative: TBK
(86) International application number: PCT/JP2019/017992
(87) International publication number: WO 2019/220936

(57) **Abstract**

A transfer sheet (10) includes a support layer (20) that is made of a porous material, and a thin-film layer (21) that is supported by the support layer and has a thickness of 5000 nm or less. The transfer sheet has a dry adhesion strength of 100 mN or more, and a wet adhesion strength of lower than the dry adhesion strength and 230 mN or less, where the dry adhesion strength is an adhesion strength between the support layer and the thin-film layer while the support layer and the thin-film layer are dry, and the wet adhesion strength is an adhesion strength between the support layer and the thin-film layer while the support layer and the thin-film layer are wet.

## Description

### [Technical Field]

The present invention relates to a transfer sheet and a method of transferring a thin-film layer using the transfer sheet.

### [Background Art]

A thin-film layer having a thickness of approximately several nm to several µm may be made of a material containing a polymer material as a main component. In recent years, adhesion of such a thin-film layer to the surface of the skin or an organ has been attempted focusing on properties of the thin-film layer such as adhesiveness to a surface of biological organs and flexibility. The thin-film layer has been reported to be usable, for example, as a dressing material for wounds (see NPL 1).

A transfer sheet is a sheet including a thin-film layer, and a support layer made of a base material for supporting the thin-film layer, and the transfer sheet is used to transfer the thin-film layer to a transfer object (see PTL 1). Specifically, the thin-film layer is transferred to the transfer object while being supported by the support layer, by pressing a surface of the thin-film layer against a target portion of the transfer object to which the thin-film layer is to be adhered, and then peeling off the support layer from the thin-film layer. Before the transfer sheet is used, the surface of the thin-film layer is covered with a protective layer made of a base material for protecting the thin-film layer (see PTL 2).

### [Citation List]

### [Patent Literature]

[PTL 1] JP 2017-19116 A
[PTL 2] JP 2015-16612 A

### [Non Patent Literature]

[NPL 1] T. Fujie et al., Adv. Funct. Mater., 2009, Vol. 19, pp. 2560-2568

### [Summary of the Invention]

### [Technical Problem]

When the thin-film layer is transferred, it is necessary to expose the surface of the thin-film layer from the protective layer and then move the sheet which is a laminate of the thin-film layer and the support layer to the target portion, and to bring the surface of the thin-film layer into contact with the target portion and then peel off the support layer from the thin-film layer. For easy handling of the transfer sheet, movement of the transfer sheet and peeling off of the support layer can preferably be performed simply. However, the thin-film layer is an extremely thin layer, and thus if an adhesion force between the thin-film layer and the support layer is excessively low, when the thin-film layer is moved to the target portion, the thin-film layer may peel peeled off from the support layer due to air currents, or wrinkling or distortion of the thin-film layer may occur due to the influence of static electricity. On the other hand, if the adhesion force between the thin-film layer and the support layer is excessively high, it is difficult to peel off the support layer from the thin-film layer.

An object of the present invention is to provide a transfer sheet that can be easily handled, and a method of transferring a thin-film layer using the transfer sheet.

### [Solution to Problem]

A transfer sheet for solving the above problem is a transfer sheet including: a thin-film layer supported by the support layer and having a thickness of 5000 nm or less, the transfer sheet having a dry adhesion strength of 100 mN or more, and a wet adhesion strength of lower than the dry adhesion strength and 230 mN or less, where the dry adhesion strength is an adhesion strength between the support layer and the thin-film layer while the support layer and the thin-film layer are dry; and the wet adhesion strength is an adhesion strength between the support layer and the thin-film layer while the support layer and the thin-film layer are wet.

According to the above configuration, when the transfer sheet in the dry state is moved to a target portion of a transfer object to which the thin-film layer is to be adhered, and the transfer sheet is wetted and then the support layer is peeled off from the thin-film layer, the transfer sheet is moved while the adhesion force is higher, and the support layer is peeled off while the adhesion force is lower. Therefore, the adhesion force can be controlled to be suitable for each of movement of the transfer sheet and peeling off of the support layer.

Since the dry adhesion strength is 100 mN or more, when the transfer sheet is moved to the target portion, it is possible to prevent peeling off of the thin-film layer from the support layer due to air current pressure or the like and the occurrence of wrinkling and distortion of the thin-film layer due to air currents, static electricity, or the like. Thus, a user of the transfer sheet can easily move the transfer sheet to the target portion. On the other hand, since the wet adhesion strength is 230 mN or less, after the transfer sheet is wetted, the support layer can be easily peeled off from the thin-film layer.

Thus, movement of the transfer sheet to the target portion and peeling off of the support layer can be simply performed, leading to easy handling of the transfer sheet.

In the above configuration, the thin-film layer may have a breaking strength of 10 mN or more.

According to the above configuration, while the support layer is peeled off from the thin-film layer, the thin-film layer is less likely to be broken, leading to a higher transfer ratio of the thin-film layer from the support layer to the transfer object.

In the above configuration, the transfer sheet may further include a protective layer, and may be configured such that the thin-film layer has a first surface in contact with the support layer and a second surface facing away from the first surface, the second surface is covered with the protective layer, and an adhesion strength between the protective layer and the thin-film layer while the protective layer and the thin-film layer are dry is 30 mN or more and less than 100 mN.

According to the above configuration, since the adhesion strength is lower than the dry adhesion strength between the support layer and the thin-film layer, when the transfer sheet is used, the protective layer can be easily peeled off from the thin-film layer. Furthermore, since the adhesion strength is 30 mN or more and less than 100 mN, the protective layer is prevented from being peeled off from the thin-film layer when not intended by the user, due to air currents or the like, and when the protective layer is to be peeled off by the user, the thin-film layer is prevented from being peeled off together with the protective layer from the support layer. Thus, it is possible to simply perform operations until completion of the transfer of the thin-film layer, that is, peeling off of the protective layer, movement of the transfer sheet to the target portion, and peeling off of the support layer. This leads to easier handling of the transfer sheet.

In the above configuration, the porous material may be a non-woven fabric.

The above configuration achieves a support layer suitable for controlling the dry adhesion strength and the wet adhesion strength between the support layer and the thin-film layer within the above respective ranges.

In the above configuration, the protective layer may be a non-woven fabric.

The above configuration achieves a protective layer suitable for controlling the adhesion strength between the protective layer and the thin-film layer within the above range.

In the above configuration, the transfer sheet may be housed in a housing made of a moisture-impermeable material.

According to the above configuration, the transfer sheet housed in the housing and stored is prevented from being wetted by moisture from the outside air.

A transfer method for solving the above problem is a method of transferring a thin-film layer using the transfer sheet, the method including: bringing a first surface of the thin-film layer into contact with a transfer object, the first surface facing away from a second surface of the thin-film layer in contact with the support layer; wetting the transfer sheet placed on the transfer object; and after wetting the transfer sheet, peeling off the support layer from the thin-film layer.

The transfer method allows transfer of the thin-film layer to the transfer object while easily handling the transfer sheet by utilizing the fact that the adhesion force between the support layer and the thin-film layer decreases due to the wetting.

### [Advantageous Effects of the Invention]

The present invention can improve ease of handling of a transfer sheet.

### [Brief Description of the Drawings]

Fig. 1 is a diagram showing a cross-sectional structure of a first form of a transfer sheet according to an embodiment.
Fig. 2 is a diagram showing a cross-sectional structure of a second form of a transfer sheet according to the embodiment.
Fig. 3 is a diagram showing a structure of a package that houses a transfer sheet according to the embodiment.
Fig. 4 is a diagram illustrating a transfer method using a transfer sheet according to the embodiment, and is a diagram showing a step of supplying a supply liquid to a transfer object.
Fig. 5 is a diagram illustrating a transfer method using a transfer sheet according to the embodiment, and is a diagram showing a step of pressing the transfer sheet against a transfer obj ect.
Fig. 6 is a diagram illustrating a transfer method using a transfer sheet according to the embodiment, and is a diagram showing a step of peeling off a support layer from a thin-film layer.

### [Description of the Embodiments]

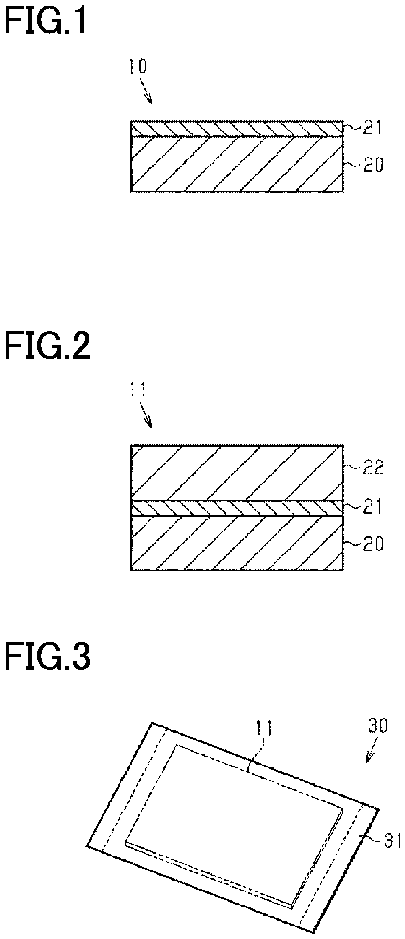

An embodiment of a transfer sheet and a method of transferring a thin-film layer will be described with reference to Figs. 1 to 6.

### [Configuration of transfer sheet]

The transfer sheet can have two forms which are a first form and a second form. These forms will be described with reference to Figs. 1 and 2.

Fig. 1 shows a transfer sheet 10 of the first form. The first form is a form of the transfer sheet when being used. As shown in Fig. 1, the transfer sheet 10 includes a support layer 20 and a thin-film layer 21. The thin-film layer 21 has two surfaces which are a first surface and a second surface, and the first surface is adhered to the support layer 20 and the second surface is exposed. The support layer 20 has a function of supporting the thin-film layer 21.

Fig. 2 shows a transfer sheet 11 of the second form. The second form is a form of the transfer sheet before being used, for example, a form of the transfer sheet while the transfer sheet is packaged. As shown in Fig. 2, in addition to the support layer 20 and the thin-film layer 21 of the first form, the transfer sheet 11 includes a protective layer 22. The protective layer 22 is in contact with the thin-film layer 21 and covers the second surface of the thin-film layer 21. In other words, the thin-film layer 21 is sandwiched between the support layer 20 and the protective layer 22. The support layer 20 and the protective layer 22 have a function of protecting the thin-film layer 21 by sandwiching the thin-film layer 21 between the layers.

By peeling off the protective layer 22 from the thin-film layer 21, the transfer sheet is transformed from the second form into the first form. It is sufficient that the transfer sheet has at least the first form.

A detailed configuration of the layers of the transfer sheets 10 and 11 will be described.

The thin-film layer 21 is thin enough to exhibit adhesiveness to a transfer object. Specifically, the thin-film layer 21 has a thickness of 1 nm or more and 5000 nm or less. The thickness of the thin-film layer 21 is preferably in the range of 100 nm or more and 750 nm or less in terms of higher adhesion to the skin, higher conformity to a surface shape of the skin, and less discomfort experienced by a living body to which the thin-film layer 21 is adhered. The thin-film layer 21 may be a layer made of a single thin film, or may be a layer made of a plurality of thin films.

The above thickness of the thin-film layer 21 is the average thickness, and is the average of thicknesses measured at a plurality of measurement points. The thickness of the thin-film layer 21 is calculated, for example, by the following method. First, samples for cross-sectional observation of the thin-film layer 21 are prepared from a plurality of portions of the transfer sheet. For example, when the transfer sheet has a rectangular shape in plan view, samples are prepared from 5 portions which are the center portion and the four corners of the transfer sheet. Then, in each of the samples, through observation of a cross section using a scanning electron microscope, the thickness is measured at 10 measurement points at 100 µm intervals, and the thicknesses are averaged to obtain the thickness of the sample. A value obtained by averaging the thicknesses of all the samples is the average thickness of the thin-film layer 21.

In the case where the thickness of the thin-film layer 21 which is thin enough to exhibit adhesiveness to the transfer object is expressed in terms of the mass of the thin-film layer 21, for example, when the thin-film layer 21 has a density in the range of 1 g/cm³ or more and 3 g/cm³ or less, the average mass of the thin-film layer 21 per unit area is in the range of 0.01 g/m² or more and 10 g/m² or less. The average mass is the mass of the thin-film layer 21 per portion of the surface of the thin-film layer 21 having an area of 1 m². When the average mass of the thin-film layer 21 is in the above range, the thin-film layer 21 itself is adhered to a surface of a living body without use of an adhesive layer.

A material for forming the thin-film layer 21 is not particularly limited as long as the material can form a thin film having the above thickness. The material of the thin-film layer 21 may be, for example, one or more materials selected from the group consisting of polymer materials such as polylactic acid, hyaluronic acid, polyglycolic acid, fibroin, polycaprolactone, and chitosan, copolymers of these polymer materials, and an acrylic urethane copolymer. The molecular weight of the material of the thin-film layer 21 is not particularly limited. The thin-film layer 21 may be made of a single type of material having a predetermined average molecular weight, or may be made of a plurality of types of materials having different average molecular weights.

The thin-film layer 21 may contain a functional material which is a material that exhibits a predetermined function in the transfer object. When the transfer object is the skin or organ of a living body, examples of the functional material include cosmetics or components of cosmetics used for skin care such as moisturizing creams and serums, dyes, drugs, proteins, and enzymes. The thin-film layer 21 may contain only a single type of functional material, or may contain two or more types of functional materials.

The support layer 20 is made of a porous material. The porous material is a base material having a large number of minute pores inside, and allows a liquid to permeate or pass through. Examples of the porous material that can be used as the support layer 20 include a sheet made of a fibrous material such as non-woven fabric, paper, knitted fabric, or woven fabric, and a resin sheet having a structure including pores such as a mesh structure. Of these base materials, a non-woven fabric is preferably used due to quick absorption and diffusion of moisture. Examples of fibers constituting the non-woven fabric include natural fibers such as cotton, hemp, wool, and pulp, semisynthetic fibers such as rayon, and synthetic fibers such as polyvinyl alcohol and polyacrylic acid. Of these fibers, natural fibers, particularly pulp is preferably used. The non-woven fabric may be made of a single type of fibers, or may be made of two or more types of fibers.

The non-woven fabric used as the support layer 20 may be a non-woven fabric manufactured by any manufacturing method, and may be, for example, a non-woven fabric manufactured by one of a spunlace method, a spunbond method, a needle punching method, a melt blow method, an air laying method, a flash spinning method, and a resin adhesion method. The non-woven fabric used as the support layer 20 preferably has a basis weight in the range of 10 g/m² or more and 150 g/m² or less, and more preferably in the range of 20 g/m² or more and 50 g/m² or less due to good feeling in use, such as texture. The basis weight of the non-woven fabric is the mass of the non-woven fabric per unit area. The thickness of the support layer 20 is not particularly limited, and selected to be, for example, in the range of 10 µm or more and 1 mm or less.

The protective layer 22 is preferably made of a porous material. The porous material for forming the protective layer 22 may be any of the materials mentioned above as the porous material for forming the support layer 20. The protective layer 22 and the support layer 20 may be made of the same type of porous material, or may be made of different types of porous materials.

The porous material for forming the protective layer 22 is also preferably a non-woven fabric. The non-woven fabric used as the support layer 20 and the non-woven fabric used as the protective layer 22 may be non-woven fabrics that are made of the same material and have the same properties such as the basis weight, or may be non-woven fabrics that differ from each other in at least one of the material and the properties.

The material of the protective layer 22 is not limited to the porous material, and may be a base material such as a resin sheet or metal foil having no pores inside. The thickness of the protective layer 22 is not particularly limited, and selected to be, for example, in the range of 10 µm or more and 1 mm or less.

### [Properties of transfer sheet]

Preferable ranges of adhesion strengths between the layers and a breaking strength of the thin-film layer 21 of the transfer sheet will be described.

First, a first dry adhesion strength Fd1 and a first wet adhesion strength Fw1 will be described. The first dry adhesion strength Fd1 is the force required to peel off the support layer 20 and the thin-film layer 21 from each other while the two layers are dry. On the other hand, the first wet adhesion strength Fw1 is the force required to peel off the support layer 20 and the thin-film layer 21 from each other while the two layers are wet.

The first dry adhesion strength Fd1 is measured by the following method. The state in which the support layer 20 and the thin-film layer 21 are dry indicates, for example, a state in which the transfer sheet is in an environment at a temperature in the range of -10°C or more and 45°C or less and a humidity in the range of 0% or more and 99% or less and in which the surface of the thin-film layer 21 is not sticky and no moisture is actively supplied to the support layer 20 or the thin-film layer 21.
1) A transfer sheet is prepared of which outer shape has been adjusted to be a circular shape with a diameter of 10 mm by a method such as cutting.
2) By using a table-top compression/tensile tester (manufactured by Shimadzu Corporation: EZ-Test, load cell: 100 N), the transfer sheet prepared at step 1) is adhered to a measurement base with a double-sided tape (manufactured by Nichiban Co., Ltd.: NICETACK sponge double-sided tape - sponge type, NW-P15) so that the thin-film layer 21 faces upward and is exposed.
3) A circular double-sided tape with a diameter of 6 mm is adhered to the center of a columnar indenter with a diameter of 8 mm, and a protective film of the circular double-sided tape is peeled off. The circular double-sided tape is prepared from the same product as the double-sided tape used at step 2).
4) The columnar indenter prepared at step 3) is pressed against the transfer sheet on the measurement base at step 2) at a compression rate of 20 mm/min and a pressure of 3 N for 3 seconds, and then the columnar indenter is vertically lifted up at a peeling speed of 10 mm /min.
5) Data on a peak strength (mN) when the thin-film layer 21 is peeled off from the support layer 20 is acquired, and the peak strength is determined as the first dry adhesion strength Fd1.

The first wet adhesion strength Fw1 is measured by the following method.

As in the method of measuring the first dry adhesion strength Fd1, steps 1) and 2) are performed. At step 2), additionally, 10 µL of water is supplied to the transfer sheet adhered to the measurement base from the periphery of the transfer sheet. Then, steps 3), 4), and 5) are sequentially performed, and the obtained peak strength (mN) is determined as the first wet adhesion strength Fw1.

In the transfer sheet of the present embodiment, the first dry adhesion strength Fd1 is 100 mN or more, and the first wet adhesion strength Fw1 is lower than the first dry adhesion strength Fd1 and is 230 mN or less.

Since the first wet adhesion strength Fw1 is lower than the first dry adhesion strength Fd1, when the transfer sheet is dry, the adhesion force between the support layer 20 and the thin-film layer 21 is higher, and when the transfer sheet is wet, the adhesion force between the support layer 20 and the thin-film layer 21 is lower. Thus, when the transfer sheet in the dry state is moved to a target portion of the transfer object to which the thin-film layer 21 is to be adhered, and the transfer sheet is wetted and then the support layer 20 is peeled off from the thin-film layer 21, the transfer sheet is moved while the adhesion force is higher, and the support layer 20 is peeled off while the adhesion force is lower. Therefore, the adhesion force can be controlled to be suitable for each of movement of the transfer sheet and peeling off of the support layer 20.

The lower adhesion strength in the wet state than in the dry state presumably occurs because the wetness of the transfer sheet causes expansion of the support layer 20 made of a porous material, entry of moisture between the support layer 20 and the thin-film layer 21, and the like.

Since the first dry adhesion strength Fd1 is 100 mN or more, sufficient adhesion force is obtained when the transfer sheet is moved to the target portion. As a result, it is possible to prevent peeling off of the thin-film layer 21 from the support layer 20 due to air current pressure or the like, and detaching of the thin-film layer 21 from the top of the support layer 20. In addition, it is possible to prevent the occurrence of wrinkling and distortion of the thin-film layer 21 due to air currents, static electricity generated between the support layer 20 and the thin-film layer 21, or the like. Thus, a user of the transfer sheet can easily move the transfer sheet to the target portion, for example, by holding the transfer sheet by hand.

On the other hand, since the first wet adhesion strength Fw1 is 230 mN or less, the adhesion force between the support layer 20 and the thin-film layer 21 in the wet state is lower than an adhesion force between the transfer object and the thin-film layer 21 expected when the transfer object is a living body, for example, an adhesion force between human skin and the thin-film layer 21. Therefore, the support layer 20 can be easily peeled off from the thin-film layer 21. Thus, when the support layer 20 is to be peeled off, the thin-film layer 21 is prevented from being peeled off together with the support layer 20 from the transfer object.

As described above, according to the transfer sheet of the present embodiment, movement of the transfer sheet to the target portion and peeling off of the support layer 20 can be simply performed, leading to easy handling of the transfer sheet.

The first dry adhesion strength Fd1 is preferably in the range of 110 mN or more and 3000 mN or less, and more preferably in the range of 350 mN or more and 600 mN or less. When the first dry adhesion strength Fd1 is the lower limit value or more, the support layer 20 and the thin-film layer 21 in the dry state are better adhered to each other, thereby more appropriately preventing peeling off of the thin-film layer 21 and the occurrence of wrinkling and distortion of the thin-film layer 21 during movement of the transfer sheet. When the first dry adhesion strength Fd1 is the upper limit value or less, the occurrence of flaws in the thin-film layer 21 is prevented.

In order to increase a transfer ratio of the thin-film layer 21 to the transfer object, the first wet adhesion strength Fw1 is preferably in the range of 50 mN or more and 200 mN or less, and more preferably in the range of 60 mN or more and 160 mN or less. The transfer ratio is a ratio of the surface area of a portion of the thin-film layer 21 transferred to the transfer object to the surface area of the thin-film layer 21 on the support layer 20.

Next, a breaking strength Fb will be described. The breaking strength Fb is the minimum force required to break the thin-film layer 21, and is measured by the following method.
1) From the transfer sheet of the second form, the protective layer 22 is peeled off, and then the support layer 20 is removed, so that the thin-film layer 21 is a single layer having a square shape with a side length of 10 mm in plan view.
2) The thin-film layer 21 obtained at step 1) is placed in a table-top compression/tensile tester (manufactured by Shimadzu Corporation: EZ-Test, load cell: 100 N).
3) A columnar indenter with a diameter of 3 mm is inserted into the thin-film layer 21 at a compression rate of 10 mm/min, and a strength (mN) when the thin-film layer 21 is broken is measured. The obtained strength is determined as the breaking strength Fb.

In the transfer sheet of the present embodiment, the breaking strength Fb is preferably 10 mN or more. When the breaking strength Fb is 10 mN or more, while the support layer 20 is peeled off from the thin-film layer 21, the thin-film layer 21 is less likely to be broken. Thus, the transfer ratio can be increased. Furthermore, the occurrence of cracking in the thin-film layer 21 adhered to the transfer object is prevented, thereby appropriately maintaining a state in which the target portion of the transfer object is covered with the thin-film layer 21.

Next, a second dry adhesion strength Fd2 will be described. The second dry adhesion strength Fd2 is the force required to peel off the protective layer 22 and the thin-film layer 21 from each other while the two layers are dry.

The second dry adhesion strength Fd2 is measured by the same method as the method of measuring the first dry adhesion strength Fd1 except that unlike the method of measuring the first dry adhesion strength Fd1, at step 2), the transfer sheet of the second form is adhered to the measurement base so that the protective layer 22 faces upward. At step 5), a peak strength (mN) when the protective layer 22 is peeled off from the thin-film layer 21 is determined as the second dry adhesion strength Fd2.

In the transfer sheet of the present embodiment, the second dry adhesion strength Fd2 is preferably lower than the first dry adhesion strength Fd1. Thus, when both the support layer 20 and the protective layer 22 are made of a porous material, the adhesion force between the porous material constituting the protective layer 22 and the thin-film layer 21 is preferably lower than the adhesion force between the porous material constituting the support layer 20 and the thin-film layer 21. In the case where the second dry adhesion strength Fd2 is lower than the first dry adhesion strength Fd1, when the transfer sheet is used, the protective layer 22 can be easily peeled off from the thin-film layer 21.

Specifically, the second dry adhesion strength Fd2 is preferably in the range of 30 mN or more and less than 100 mN. When the second dry adhesion strength Fd2 is 30 mN or more, the protective layer 22 is prevented from being peeled off from the thin-film layer 21 when not intended by the user, due to air currents or the like. Even if the protective layer 22 is peeled off from the thin-film layer 21 at an unintended timing, the thin-film layer 21 can be transferred by using the transfer sheet which is a laminate of the thin-film layer 21 and the support layer 20. However, in order to maintain quality of the thin-film layer 21 and prevent adhesion of dust or the like to the thin-film layer 21, the thin-film layer 21 is preferably covered with the protective layer 22 until the protective layer 22 is intentionally peeled off by the user.

In the case where the second dry adhesion strength Fd2 is less than 100 mN, when the protective layer 22 is to be peeled off by the user, the thin-film layer 21 is prevented from being peeled off together with the protective layer 22 from the support layer 20. Therefore, the protective layer 22 can be easily peeled off from the thin-film layer 21. In the case where the second dry adhesion strength Fd2 is in the range of 30 mN or more and less than 100 mN and the first dry adhesion strength Fd1 is 100 mN or more, while the protective layer 22 is peeled off, the thin-film layer 21 is prevented from being peeled off from both the protective layer 22 and the support layer 20 due to the first dry adhesion strength Fd1 being as low as the second dry adhesion strength Fd2.

The second dry adhesion strength Fd2 is preferably in the range of 45 mN or more and 95 mN or less, and more preferably in the range of 60 mN or more and 90 mN or less. When the second dry adhesion strength Fd2 is the lower limit value or more, the protective layer 22 is more appropriately prevented from being peeled off from the thin-film layer 21 when not intended by the user. When the second dry adhesion strength Fd2 is the upper limit value or less, the protective layer 22 is more easily peeled off from the thin-film layer 21. When the second dry adhesion strength Fd2 is in the range of 45 mN or more and 95 mN or less, the magnitude of the adhesion force between the protective layer 22 and the thin-film layer 21 is suitable for picking up the protective layer 22 with the fingers and peeling off the protective layer 22 by the user. When the second dry adhesion strength Fd2 is in the range of 60 mN or more and 90 mN or less, the magnitude of the adhesion force is more suitable for picking up the protective layer 22 with the fingers and peeling off the protective layer 22 by the user.

As described above, by controlling the adhesion strengths Fd1, Fw1, and Fd2, it is possible to simply perform operations from extraction of the transfer sheet from the package by the user until completion of the transfer of the thin-film layer 21, that is, peeling off of the protective layer 22, movement of the transfer sheet to the target portion, and peeling off of the support layer 20. This leads to easier handling of the transfer sheet.

### [Method of manufacturing transfer sheet]

An example of a method of manufacturing a transfer sheet will be described. The transfer sheet may be manufactured by a method different from the following manufacturing method as long as the transfer sheet has a first dry adhesion strength Fd1 of 100 mN or more and a first wet adhesion strength Fw1 of 230 mN or less.

First, the thin-film layer 21 is formed on a surface of a film forming material. The film forming material may be a resin sheet made of a thermoplastic resin, a thermosetting resin, a watersoluble resin, or the like.

Specifically, a coating liquid in which a material of the thin-film layer 21 is dissolved is applied to the surface of the film forming material to form a coating film, and the coating film is dried to form the thin-film layer 21. A solvent of the coating liquid may be a protic polar solvent or an aprotic polar solvent depending on properties of the material of the thin-film layer 21. Examples of a protic polar solvent include water, ethanol, isopropanol, and acetic acid. Examples of an aprotic polar solvent include ethyl acetate, butyl acetate, propyl acetate, methyl ethyl ketone, acetone, and dimethyl sulfoxide.

The method of applying the coating liquid is not particularly limited as long as a coating film having a desired thickness can be formed by the method. The application method is preferably, for example, one of a gravure method, a micro gravure method, a spin coating method, a spray coating method, and an electrospinning method. The drying temperature of the coating film is preferably a boiling point of the solvent of the coating liquid or higher.

Subsequently, a porous material used as the support layer 20 is brought into contact with a surface of the thin-film layer 21 on the film forming material, and the thin-film layer 21 is transferred from the film forming material to the support layer 20. The transfer method may be a known transfer method such as a method using peeling off by suction or a method using a sacrificial film.

Next, the protective layer 22 is laminated on a surface of the thin-film layer 21 of a laminate of the support layer 20 and the thin-film layer 21, that is, a surface of the thin-film layer 21 on a side facing away from the surface of the thin-film layer 21 in contact with the support layer 20. Thus, the transfer sheet 11 of the second form is formed. The outer shape of the transfer sheet 11 may be adjusted to be a desired shape by a method such as cutting out as needed.

Desired first dry adhesion strength Fd1, first wet adhesion strength Fw1, and breaking strength Fb can be obtained by adjusting the material and thickness of the thin-film layer 21, the surface roughness of the film forming material, the application speed of the coating film during film formation, the drying temperature of the coating film during film formation, the material of the support layer 20, the basis weight of the support layer 20, the degree of change in the shape of the support layer 20 due to the wetting, conditions for the transfer of the thin-film layer 21 from the film forming material to the support layer 20, and the like.

The second dry adhesion strength Fd2 can be changed by adjusting the material and thickness of the thin-film layer 21, the material of the protective layer 22, the magnitude of the pressure applied to the protective layer 22 when the protective layer 22 is laminated on the thin-film layer 21, and the like.

### [Package]

Fig. 3 shows a package in which the transfer sheet is housed. A package 30 includes the transfer sheet 11 of the second form, and a housing 31 that houses the transfer sheet 11. The housing 31 has any shape that can house the transfer sheet 11, including a bag shape or a box shape.

The housing 31 is preferably configured to be sealable, and is preferably made of a moisture-impermeable material. The moisture-impermeable material is a material having a moisture permeability measured according to JIS Z 0208 of 5 g/m²/24h or less.

Examples of the material for forming the housing 31 include aluminum and nylon. The housing 31 is preferably made of aluminum in terms of low moisture permeability. The housing 31 preferably, for example, has a bag shape made of an aluminum sheet.

Since the transfer sheet 11 is housed in the housing 31 made of a moisture-impermeable material, the transfer sheet 11 is prevented from being wetted by moisture from the outside air. Thus, the transfer sheet 11 can be stored as the package 30 in an environment at normal temperature and humidity.

### [Method of transferring thin-film layer]

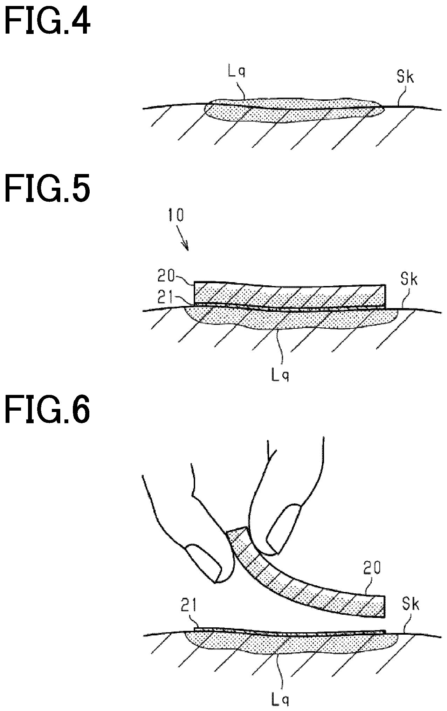

A method of using the transfer sheet, that is, a method of transferring a thin-film layer by using the transfer sheet will be described with reference to Figs. 4 to 6.

As shown in Fig. 4, first, a supply liquid Lq is supplied to an target portion of a transfer object Sk to which the thin-film layer 21 is to be adhered. The transfer sheet of the present embodiment is suitable for transfer of the thin-film layer to a living body such as a surface of the skin or organ, and in particular, preferably used for transfer of the thin-film layer to human skin. The supply liquid Lq only needs to be a liquid that can wet the thin-film layer 21 and the support layer 20, and may be specifically a liquid containing water, or an oil such as massage oil. When the transfer object Sk is human skin, the supply liquid Lq may be, for example, water, or cosmetics such as skin lotion.

Subsequently, the transfer sheet 11 of the second form is extracted out from the package 30 and the protective layer 22 is peeled off from the transfer sheet 11 to obtain the transfer sheet 10 of the first form. Then, as shown in Fig. 5, the transfer sheet 10 is placed on the transfer object Sk so that the surface of the thin-film layer 21 is in contact with the target portion of the transfer object Sk. The transfer sheet 10 is pressed with a finger or the like from above the support layer 20 so that the supply liquid Lq permeates the support layer 20. The load applied to the transfer sheet 10 while the transfer sheet 10 is pressed is preferably in the range of 10 g/cm² or more and 900 g/cm² or less. When the transfer object Sk is human skin, the load is preferably in the range of 10 g/cm² or more and 20 g/cm² or less due to less damage to the skin. By the application of such a load, the thin-film layer 21 is sufficiently adhered to the transfer object Sk.

As shown in Fig. 6, then, the support layer 20 is peeled off from the thin-film layer 21. Thus, the thin-film layer 21 is transferred to the transfer object Sk. This transfer method allows transfer of the thin-film layer 21 to the transfer object Sk while easily handling the transfer sheet by utilizing the fact that the adhesion force between the support layer 20 and the thin-film layer 21 decreases due to the wetting.

In the above transfer method, the supply liquid Lq is supplied to the transfer object Sk before the transfer sheet 10 is placed on the transfer object Sk. However, the supply liquid Lq may be supplied to the transfer sheet 10 after the transfer sheet 10 is placed on the transfer object Sk. In other words, the transfer sheet 10 placed on the transfer object Sk only needs to be wetted.

### [Examples]

The aforementioned transfer sheet will be described by way of specific examples.

### <Manufacture of transfer sheet>

DL-polylactic acid (manufactured by Musashino Chemical Laboratory, Ltd.) was dissolved in ethyl acetate to generate a coating liquid for forming a thin-film layer. The amount of polylactic acid in the coating liquid was adjusted so that the coating liquid had a solid content in the range of 5% or more and 10% or less. The average molecular weight of polylactic acid was selected to be in the range of 50,000 or more and 440,000 or less. The coating liquid was applied to a PET sheet (manufactured by Toray Industries, Inc.: Lumirror, S10) as a film forming material by using a wire bar to form a coating film. The coating film was heated in an oven to be dried and solidified to form a thin-film layer. The thin-film layer was formed to have a thickness after drying in the range of 100 nm or more and 1200 nm or less. The heating temperature during drying was selected to be in the range of 70°C or more and 120°C or less.

Subsequently, a non-woven fabric (manufactured by Futamura Chemical Co., Ltd.) as a support layer was laminated on the thin-film layer, the film forming material was peeled off, and the thin-film layer was transferred from the film forming material to the support layer. The non-woven fabric contained cellulose as a main component, and had a basis weight of 20 g/m².

A surface of the thin-film layer on a side facing away from the support layer was covered with a non-woven fabric as a protective layer. Then, the non-woven fabric was cut into a desired size by using a trimming cutter to obtain a transfer sheet of Examples 1 to 18 and Comparative Example. The protective layer was a non-woven fabric of the same type as the support layer.

In the above manufacturing method, the surface roughness of the film forming material, the thickness of the thin-film layer, and the basis weight of the non-woven fabric as the support layer were changed to cause the first dry adhesion strength Fd1 to be different in Examples 1 to 18 and Comparative Example.

Specifically, when the surface roughness of the film forming material is high, that is, the film forming material has unevenness on the surface thereof, as the thickness of the thin-film layer decreases, the unevenness of the film forming material is less likely to be smoothed by the coating film to be the thin-film layer, and the unevenness of the film forming material is more likely to be reflected in the surface shape of the thin-film layer. That is, as the thickness of the thin-film layer decreases, the unevenness of the surface of the thin-film layer in contact with the support layer increases. As a result, the area of a portion of the thin-film layer in contact with the support layer decreases, and thus the first dry adhesion strength Fd1 between the thin-film layer and the support layer decreases. On the other hand, when the film forming material has unevenness on the surface thereof, as the thickness of the thin-film layer increases, the unevenness of the film forming material is better smoothed by the coating film to be the thin-film layer, and the surface of the thin-film layer in contact with the support layer is more flattened. As a result, the area of a portion of the thin-film layer in contact with the support layer increases, and thus the first dry adhesion strength Fd1 between the thin-film layer and the support layer increases.

As the basis weight of the non-woven fabric as the support layer increases, the number of fibers per unit area increases, and thus the area of a portion of the support layer in contact with the thin-film layer increases. As a result, the first dry adhesion strength Fd1 between the thin-film layer and the support layer increases.

In the above manufacturing method, the thickness of the thin-film layer was changed to cause the breaking strength Fb to be different in Examples 1 to 18 and Comparative Example. Specifically, as the thickness of the thin-film layer increases, the breaking strength Fb increases.

In the above manufacturing method, the degree of change in the shape of the support layer due to the wetting was changed to cause the first wet adhesion strength Fw1 to be different in Examples 1 to 18 and Comparative Example. As the change in the shape of the support layer due to the wetting increases, in other words, as a swelling ratio of the support layer increases, a larger gap is generated between the thin-film layer and the support layer in the wet state and the area of a portion at which the thin-film layer is adhered to the support layer decreases, and thus the first wet adhesion strength Fw1 decreases. The degree of change in the shape of the support layer due to the wetting can be changed by adjusting the material, basis weight, and water absorption ratio of the non-woven fabric used as the support layer.

In the above manufacturing method, the magnitude of the pressure applied when the protective layer was laminated on the thin-film layer was changed to cause the second dry adhesion strength Fd2 to be different in Examples 1 to 18 and Comparative Example. As the magnitude of the pressure increases, the second dry adhesion strength Fd2 increases.

In the examples and the comparative example, the first dry adhesion strength Fd1, the first wet adhesion strength Fw1, the breaking strength Fb, and the second dry adhesion strength Fd2 were measured according to the measuring methods described in the above embodiment.

### <Evaluation method>

In the examples and the comparative example, an outer shape of the transfer sheet was adjusted to be a 30 mm × 40 mm rectangle, and the transfer sheet was evaluated for the following items.

### (Peelability of protective layer)

In the examples and the comparative example, the protective layer was picked up with the fingers and peeled off from the thin-film layer. The peelability was evaluated as "Good" when the protective layer that covered the thin-film layer was completely peeled off while the thin-film layer was not peeled off together with the protective layer from the support layer. The peelability was evaluated as "Poor" when part of the thin-film layer remained attached to the protective layer when peeled off from the support layer, or when the protective layer was not adhered to the thin-film layer and before being picked up with the fingers, the protective layer was unintentionally peeled off partially or entirely due to air currents or the like.

### (Handleability)

In the examples and the comparative example, the transfer sheet from which the protective layer had been peeled off was held by hand and moved up and down and left and right. Then, it was visually checked whether the thin-film layer was peeled off from the support layer and whether wrinkling or distortion occurred in the thin-film layer. The handleability was evaluated as "Good" when the thin-film layer was not peeled off from the support layer and no wrinkling or distortion occurred in the thin-film layer. The handleability was evaluated as "Poor" when the thin-film layer was partially or entirely peeled off from the support layer, or when wrinkling or distortion occurred in the thin-film layer.

### (Transfer ratio)

As a supply liquid, 250 µL of water was supplied to human skin which was a transfer object, and the water was lightly spread with a finger. Then, in the examples and the comparative example, the transfer sheet from which the protective layer had been peeled off was placed on the skin so that the thin-film layer was in contact with the skin. Subsequently, the transfer sheet on the skin was covered with a non-woven fabric different from the transfer sheet, and the transfer sheet was pressed against the skin from above the non-woven fabric for 3 seconds with a force that caused no pain, followed by removal of the non-woven fabric. Then, the support layer was gradually peeled off with the fingers from a corner of the transfer sheet. After the support layer was peeled off, the thin-film layer on the skin was visually observed, and the surface area of the thin-film layer was obtained. The obtained surface area of the thin-film layer was compared with the surface area of the thin-film layer of the transfer sheet before the transfer of the thin-film layer to calculate a transfer ratio.

### <Evaluation results>

Table 1 shows the evaluation results of the first dry adhesion strength Fd1, the first wet adhesion strength Fw1, the breaking strength Fb, the handleability, and the transfer ratio in the examples and the comparative example. In the comparative example, the transfer sheet was not kept taut in the wet state, and thus the first wet adhesion strength Fw1 was not able to be measured. Table 2 shows the evaluation results of the second dry adhesion strength Fd2 and the peelability of the protective layer in the examples and the comparative example. Tables 1 and 2 show the values of the adhesion strengths and the breaking strength, rounding off the first decimal place.

**[Table 1]**

| | First dry adhesion strength Fd1 (mN) | First wet adhesion strength Fw1 (mN) | Breaking strength Fb (mN) | Evaluation results | |
|---|---|---|---|---|---|
| | | | | Transfer ratio (%) | Handleability |
| Example 1 | 119 | 110 | 46 | 100 | Good |
| Example 2 | 135 | 72 | 51 | 100 | Good |
| Example 3 | 145 | 108 | 21 | 100 | Good |
| Example 4 | 398 | 110 | 22 | 100 | Good |
| Example 5 | 340 | 95 | 60 | 100 | Good |
| Example 6 | 392 | 196 | 78 | 100 | Good |
| Example 7 | 446 | 95 | 32 | 100 | Good |
| Example 8 | 460 | 132 | 80 | 100 | Good |
| Example 9 | 511 | 179 | 56 | 100 | Good |
| Example 10 | 512 | 93 | 16 | 100 | Good |
| Example 11 | 538 | 173 | 84 | 100 | Good |
| Example 12 | 592 | 70 | 23 | 100 | Good |
| Example 13 | 596 | 207 | 70 | 100 | Good |
| Example 14 | 599 | 173 | 53 | 100 | Good |
| Example 15 | 600 | 167 | 55 | 100 | Good |
| Example 16 | 603 | 125 | 28 | 100 | Good |
| Example 17 | 1168 | 193 | 7 | 95 | Good |
| Example 18 | 1223 | 146 | 0 | 80 | Good |
| Comparative Example | 67 | - | 140 | 95 | Poor |

**[Table 2]**

| | Second dry adhesion strength Fd2 (mN) | Peelability |
|---|---|---|
| Example 1 | 87 | Good |
| Example 2 | 41 | Good |
| Example 3 | 37 | Good |
| Example 4 | 84 | Good |
| Example 5 | 71 | Good |
| Example 6 | 71 | Good |
| Example 7 | 37 | Good |
| Example 8 | 87 | Good |
| Example 9 | 71 | Good |
| Example 10 | 87 | Good |
| Example 11 | 84 | Good |
| Example 12 | 41 | Good |
| Example 13 | 87 | Good |
| Example 14 | 84 | Good |
| Example 15 | 71 | Good |
| Example 16 | 71 | Good |
| Example 17 | 84 | Good |
| Example 18 | 87 | Good |
| Comparative Example | 10 | Poor |

As shown in Table 1, in Examples 1 to 18 in which the first dry adhesion strength Fd1 was 100 mN or more and the first wet adhesion strength Fw1 was 230 mN or less, the handleability was good, and a good transfer ratio of 80% or more was obtained. On the other hand, in Comparative Example in which the first dry adhesion strength Fd1 was less than 100 mN, when the transfer sheet was moved up and down and left and right, the thin-film layer was peeled off from the support layer and wrinkling occurred in the thin-film layer, and thus the handleability was poor.

Thus, it was found that when the first dry adhesion strength Fd1 is 100 mN or more and the first wet adhesion strength Fw1 is 230 mN or less, the transfer sheet can be easily handled.

In Examples 1 to 16 in which the breaking strength Fb was 10 mN or more, a higher transfer ratio was obtained than in Examples 17 and 18 in which the breaking strength Fb was less than 10 mN. In Examples 1 to 16, the transfer ratio was 100%, that is, the entire thin-film layer was transferred to the transfer object. Thus, it was found that when the breaking strength Fb is 10 mN or more, a particularly high transfer ratio is obtained.

As shown in Table 2, in all of Examples 1 to 18 in which the second dry adhesion strength Fd2 was in the range of 30 mN or more and less than 100 mN, the protective layer had good peelability. On the other hand, in Comparative Example in which the second dry adhesion strength Fd2 was less than 30 mN, the protective layer was peeled off due to air current pressure before being picked up with the fingers.

Thus, it was found that when the second dry adhesion strength Fd2 is in the range of 30 mN or more and less than 100 mN, the protective layer can be easily peeled off.

As described in the embodiment and the examples, the transfer sheet and the method of transferring a thin-film layer according to the embodiment achieves the following effects.
(1) The first dry adhesion strength Fd1 is 100 mN or more, and the first wet adhesion strength Fw1 is lower than the first dry adhesion strength Fd1 and is 230 mN or less. Thus, the transfer sheet in the dry state can be easily moved to the target portion, and after the transfer sheet is wetted, the support layer 20 can be easily peeled off from the thin-film layer 21. Therefore, the transfer sheet can be easily handled.
(2) The breaking strength Fb of the thin-film layer 21 is 10 mN or more. Thus, while the support layer 20 is peeled off from the thin-film layer 21, the thin-film layer 21 is less likely to be broken, leading to a higher transfer ratio of the thin-film layer 21 from the support layer 20 to the transfer obj ect.
(3) The second dry adhesion strength Fd2 is in the range of 30 mN or more and less than 100 mN. Thus, when the transfer sheet is used, the protective layer 22 can be easily peeled off from the thin-film layer 21.
(4) The support layer 20 made of a non-woven fabric provides a support layer 20 suitable for controlling the first dry adhesion strength Fd1 and the first wet adhesion strength Fw1 within the above respective ranges. Furthermore, the protective layer 22 made of a non-woven fabric provides a protective layer 22 suitable for controlling the second dry adhesion strength Fd2 within the above range.
(5) Since the transfer sheet is housed in the housing 31 made of a moisture-impermeable material, the transfer sheet housed in the housing 31 and stored is prevented from being wetted by moisture from the outside air.
(6) The method of transferring a thin-film layer includes bringing a first surface of the thin-film layer 21 into contact with the transfer object, the first surface facing away from a second surface of the thin-film layer 21 in contact with the support layer 20, wetting the transfer sheet placed on the transfer object, and after wetting the transfer sheet, peeling off the support layer 20 from the thin-film layer 21. This transfer method allows transfer of the thin-film layer 21 to the transfer object while easily handling the transfer sheet by utilizing the fact that the adhesion force between the support layer 20 and the thin-film layer 21 decreases due to the wetting.

### [Reference Signs List]

- Lq: Supply liquid
- Sk: Transfer object
- 10, 11: Transfer sheet
- 20: Support layer
- 21: Thin-film layer
- 22: Protective layer
- 30: Package
- 31: Housing

## Claims

1. A transfer sheet comprising:
a support layer made of a porous material; and
a thin-film layer supported by the support layer and having a thickness of 5000 nm or less,
the transfer sheet having a dry adhesion strength of 100 mN or more, and a wet adhesion strength of lower than the dry adhesion strength and 230 mN or less, where
the dry adhesion strength is an adhesion strength between the support layer and the thin-film layer while the support layer and the thin-film layer are dry; and
the wet adhesion strength is an adhesion strength between the support layer and the thin-film layer while the support layer and the thin-film layer are wet.

2. The transfer sheet according to claim 1, wherein the thin-film layer has a breaking strength of 10 mN or more.

3. The transfer sheet according to claim 1 or 2, further comprising a protective layer, wherein:
the thin-film layer has a first surface in contact with the support layer and a second surface facing away from the first surface;
the second surface is covered with the protective layer; and
an adhesion strength between the protective layer and the thin-film layer while the protective layer and the thin-film layer are dry is 30 mN or more and less than 100 mN.

4. The transfer sheet according to any one of claims 1 to 3, wherein the porous material is a non-woven fabric.

5. The transfer sheet according to claim 3, wherein the protective layer is a non-woven fabric.

6. The transfer sheet according to any one of claims 1 to 5, the transfer sheet being housed in a housing made of a moisture-impermeable material.

7. A method of transferring a thin-film layer using the transfer sheet according to any one of claims 1 to 6, the method comprising:
bringing a first surface of the thin-film layer into contact with a transfer object, the first surface facing away from a second surface of the thin-film layer in contact with the support layer;
wetting the transfer sheet placed on the transfer object; and
after wetting the transfer sheet, peeling off the support layer from the thin-film layer.
